(19) Europäisches Patentamt
European Patent Office
Office européen des brevets

(11) EP 0 581 115 B1

(12) **EUROPÄISCHE PATENTSCHRIFT**

(45) Veröffentlichungstag und Bekanntmachung des Hinweises auf die Patenterteilung:
**15.05.1996 Patentblatt 1996/20**

(51) Int. Cl.$^6$: **C07C 68/08**, C07C 69/96

(21) Anmeldenummer: **93111280.9**

(22) Anmeldetag: **14.07.1993**

(54) **Verfahren zur Abtrennung von Methanol aus Dimethylcarbonat/Methanol-Gemischen**

Process for separating methanol from dimethyl carbonate/methanol mixtures

Procédé de séparation du méthanol de mélanges carbonate de diméthyle/méthanol

(84) Benannte Vertragsstaaten:
**BE DE ES FR GB IT NL**

(30) Priorität: **27.07.1992 DE 4224770**

(43) Veröffentlichungstag der Anmeldung:
**02.02.1994 Patentblatt 1994/05**

(73) Patentinhaber: **BAYER AG**
**D-51368 Leverkusen (DE)**

(72) Erfinder:
• **Mais, Franz-Josef, Dr.**
**D-40215 Düsseldorf (DE)**
• **Wagner, Paul, Dr.**
**D-40597 Düsseldorf (DE)**
• **Buysch, Hans-Josef, Dr.**
**D-47809 Krefeld (DE)**

(56) Entgegenhaltungen:
**EP-A- 0 000 894         EP-A- 0 001 780**

**Beschreibung**

Die Erfindung betrifft ein Verfahren zur Abtrennung von Methanol aus Dimethylcarbonat (DMC) und Methanol enthaltenden Gemischen durch Azeotrop-Destillation mit einem Schleppmittel bevorzugt bei Normaldruck. Das erhaltene Azeotrop wird durch Zusatz von Wasser aufgetrennt, das dabei erhaltene Schleppmittel wird in die Azeotrop-Destillation zurückgeführt, und aus der wäßrig-methanolischen Phase wird das Methanol destillativ gewonnen. Aus dem Sumpf der Azeotrop-Destillation kann sodann das DMC gewonnen werden.

DMC ist ein wertvolles Zwischenprodukt zur Herstellung aromatischer Carbonate. Diese Carbonate und DMC selbst sind ihrerseits Vorprodukte zur Herstellung von Pflanzenschutzmitteln, Pharmazeutika, Farbstoffen und Kunststoffen, wie beispielsweise Polycarbonaten (Ullmanns Encyclopädie der technischen Chemie, 3. Auflage, Band 9, S. 776). DMC ist darüber hinaus ein wertvolles Lösungsmittel.

Bei der chlorvermeidenden Herstellung von DMC fällt dies üblicherweise im Gemisch mit Methanol an. Dies gilt beispielsweise für die Herstellung von DMC aus Kohlenmonoxid, Methanol und Sauerstoff in Gegenwart von beispielsweise CuCl (DE-OS 21 10 194; US 3.980.690) und beispielsweise für die Herstellung aus Kohlenmonoxid und Methylnitrit in Gegenwart von Katalysatoren (etwa Beispiel 1 von EP 425 197) und weiterhin beispielsweise für die Herstellung von DMC durch Alkoholyse von Ethylenglykolcarbonat mit Methanol (US 3.642.858; EP 298 167).

Bei der Normaldruckdestillation des Produktgemisches aus DMC und Methanol zur Rückführung des Methanols und zur Reinigung des DMC gewinnt man ein Azeotrop aus etwa 30 Gew.-% DMC und etwa 70 Gew.-% Methanol. Mit dieser Methode ist eine einfache Trennung nicht möglich.

Daher sind zur Auftrennung eines solchen Gemisches verschiedene Verfahren erarbeitet worden. So wird in US 3.803.201 vorgeschlagen, durch Kühlung des genannten azeotropen Gemisches auf -70°C eine mit DMC stark angereicherte Fraktion auszufrieren. Das Verfahren ist jedoch angesichts der erforderlichen niedrigen Temperatur sehr aufwendig und daher technisch nicht brauchbar. In US 3.963.586 wird vorgeschlagen, durch eine Extraktivdestillation unter Verwendung von Wasser eine Methanolabtrennung zu erreichen. In DE-OS 27 06 684 werden als Extraktivlösungsmittel organische Verbindungen, wie Methylglykolacetat, Chlorbenzol, Butyrolacton oder Ethylenglykolcarbonat vorgeschlagen. Diese Verfahren sind jedoch wegen der hohen Mengen an Extraktivlösungsmittel sehr ungünstig; so wird beispielsweise gemäß US' 586 die 20-fache Menge an Wasser, bezogen auf das Einsatzgemisch, benötigt.

In DE-OS 26 07 003 und JP 02/212 456 wird vorgeschlagen, das Gemisch durch Destillation unter Druck zu trennen. Aber auch hierbei enthält das erhaltene Destillat noch erhebliche Mengen an DMC, beispielsweise gemäß JP' 456 noch 17,5 Gew.-% (dortiges Beispiel 1). Damit wird eine zweite, diesmal unter Normaldruck oder Vakuum durchgeführte Destillation zur Abtrennung des DMC in Form des DMC/Methanol- Azeotrops aus dem angereicherten Methanol notwendig. Insgesamt müssen bei solchen Verfahren in den erforderlichen Kreisströmen große Substanzmengen bewältigt werden, was einen hohen Energieaufwand erfordert.

In US 4.582.645 wird ein Verfahren zur Trennung eines DMC/Methanol-Gemisches durch Kontaktieren dieses Gemisches mit hydrophoben Zeolithen beschrieben, beispielsweise mit Silicaliten, ZSM 5 oder NU- bzw. EU-Zeolithtypen. Nach den Beispielen dieser US' 645 eignet sich das beschriebene Verfahren lediglich zur Entfernung kleiner Restgehalte an DMC aus Methanol, da man hohe Gewichtsmengen an Zeolith, bezogen auf DMC, anwenden muß.

In US 4.798. 674 und EP 423 949 wird ein Verfahren zur Trennung von DMC/Methanol-Gemischen mittels spezieller Membranen beschrieben. Eine vollständige Trennung ist jedoch nicht möglich. Im Methanol-Permeatstrom sind immer noch DMC-Restgehalte im Prozentbereich enthalten.

In EP 894, EP 1 780 und JP 63/205 101 (1988) wird vorgeschlagen, das DMC/Methanol-Gemisch durch Zusatz eines azeotropbildenden Kohlenwasserstoffs, beispielsweise Hexan, Heptan, Cyclohexan oder Benzol, zu trennen. In den beiden erstgenannten Anmeldungen wird weiterhin beschrieben, daß sich im Anschluß an diese Azeotrop-Destillation das Destillat in zwei Phasen trennt, nämlich in die obere Kohlenwasserstoffphase und die untere Methanolphase. In eigenen Versuchen (siehe Vergleichsbeispiele) wurde jedoch gefunden, daß diese Phasentrennungen nur sehr unvollkommen erfolgen und daß beide Phasen lediglich unterschiedlich zusammengesetzte Gemische aus allen drei Stoffen, nämlich dem Schleppmittel, Methanol und DMC darstellen. Eine notwendige vollständige Trennung führt daher zu sehr großen Kreisströmen. Dies ist technisch ausgesprochen ungünstig, da ebenso wie bei der Druckdestillation durch die großen Kreisströme ein erheblicher Energieaufwand erforderlich ist.

Es wurde nun ein Verfahren zur Abtrennung von Methanol aus Dimethylcarbonat/Methanol-Gemischen durch Azeotrop-Destillation mit einem Schleppmittel bei Normaldruck gefunden, das dadurch gekennzeichnet ist, daß

a) ein mit Methanol ein Azeotrop bildendes Schleppmittel eingesetzt wird, das mit Wasser nicht mischbar ist,

b) das Azeotrop dieses Schleppmittels mit Methanol bei tieferer Temperatur als das Azeotrop Dimethylcarbonat/Methanol siedet,

c) das Azeotrop Schleppmittel/Methanol, gegebenenfalls geringe Mengen Dimethylcarbonat enthaltend, mit Wasser zur Bildung zweier Phasen versetzt wird,

d) die gebildeten Phasen getrennt werden und die Schleppmittelphase, die geringe Mengen an Methanol und Dimethylcarbonat enthalten kann, in die Destillationskolonne zurückgeführt und die wäßrig-methanolische Phase, die geringe Mengen an Dimethylcarbonat und Schleppmittel enthalten kann, durch Destillation auf Methanol und Dimethylcarbonat/Schleppmittel aufgearbeitet werden und

e) aus dem Methanol-freien Sumpf der Azeotrop-Destillation das Dimethylcarbonat gewonnen wird.

Das erfindungsgemäße Verfahren ermöglicht die Trennung von DMC- und Methanol-haltigen Gemischen, aus denen ohne Schleppmittelzusatz lediglich ein DMC/Methanol-Azeotrop der ungefähren Zusammensetzung von 30 Gew.-% DMC und 70 Gew.-% Methanol abdestilliert. Solche Gemische können weitere Bestandteile enthalten, deren Siedepunkte über dem des genannten Azeotrops liegt. Ein solcher weiterer Bestandteil kann insbesondere Wasser sein, das bei der Herstellung des DMC aus Methanol, Kohlenmonoxid und Sauerstoff im Produktgemisch anwesend ist. Es ist weiterhin möglich, daß die zu trennenden Gemische gelöste Feststoffe, wie beispielsweise Katalysatorreste, enthalten.

Erfindungsgemäß wird diesem Gemisch ein Schleppmittel zugesetzt, das mit Methanol ein Azeotrop bildet. Kennzeichnend für die vorliegende Erfindung ist es, daß der Siedepunkt des Azeotrops aus Methanol und Schleppmittel niedriger ist als der Siedepunkt des Azeotrops aus Methanol und DMC und daß das Schleppmittel mit Wasser nicht mischbar ist. Die erfindungsgemäß durchzuführende Destillation wird dann üblicherweise bei Normaldruck durchgeführt. Es ist selbstverständlich auch möglich, bei erhöhtem oder erniedrigtem Druck zu destillieren, falls bei einem solchen vom Normaldruck abweichenden Druck das Azeotrop aus Methanol und Schleppmittel erhalten bleibt oder sogar stärker ausgeprägt ist. Ein Abweichung vom Normaldruck bringt im allgemeinen aber keine Vorteile, sondern wegen der aufwendigeren Apparatur lediglich zusätzliche Kosten.

Die erfindungsgemäß einsetzbaren Schleppmittel, die den beiden obigen Bedingungen - nicht mischbar mit Wasser und Siedepunkt des Azeotrops Schleppmittel/Methanol tiefer als der des Azeotrops DMC/Methanol - finden sich in vielen unterschiedlichen chemischen Stoffklassen. Bevorzugte Stoffklassen sind geradkettige oder verzweigte gesättigte Kohlenwasserstoffe, beispielsweise n-Pentan, n-Hexan, n-Heptan, n-Octan und deren verzweigte Isomere; gesättigte cyclische $C_5$-$C_8$-Kohlenwasserstoffe, wie Cyclopentan, Cyclohexan, Methylcyclopentan, Ethylcyclobutan, Cycloheptan, Methylcyclohexan, Ethylcyclopentan, Propylcyclobutan; ungesättigte, geradkettige oder verzweigte $C_5$-$C_8$-Kohlenwasserstoffe, wie n-Penten, n-Hexen, n-Hepten, n-Octen, sowie deren verzweigte Isomere und alle Isomere, die sich durch die verschiedene Stellung der Doppelbindung ergeben; Benzol; halogenierte $C_2$-$C_4$-Kohlenwasserstoffe, wie 1,1,1-Trichlorethan, 1,2-Dichlorethan, 1,2-Dichlor-1-propen, 1-Brompropan, 1-Chlorbutan, 2-Brom-2-methylpropan, 1-Chlor-3-methylbutan, 1,1,2-Trichloro-trifluoroethan; Fluorbenzol; offenkettige oder cyclische, gesättigte oder ungesättigte $C_4$-$C_8$-Ether und -Thioether, wie Butylmethyl-ether, Methyl-tert.-butylether, Diisopropylether, Furan, 2-Methylfuran, 2,5-Dimethylfuran, Thiophen; $C_4$-$C_8$-Ester, wie Propylformiat, Isopropylformiat, Methylpropionat; und andere, die sich für den Fachmann in naheliegender Weise ergeben.

Das Schleppmittel wird in einer Menge von 30 bis 300 Gew.-%, bezogen auf das abzutrennende Methanol, eingesetzt.

Bei der erfindungsgemäß durchzuführenden Azeotrop-Destillation fällt als Kopfprodukt ein Gemisch aus Methanol, Schleppmittel und gegebenenfalls kleinen Mengen an Dimethylcarbonat an. Für den Fall, daß das zu trennende Gemisch auch untergeordnete Mengen Wasser enthielt, findet sich dieses Wasser auch im Kopfprodukt. Als Bodenprodukt erhält man reines DMC, das für den Fall hereingeschleppter gelöster Feststoffe, beispielsweise Katalysatorreste, auch noch diese Feststoffe enthält.

Es ist ein weiteres Merkmal des erfindungsgemäßen Verfahrens, daß dem genannten Kopfprodukt Wasser zugesetzt wird, welches eine Trennung des Kopfproduktes in zwei Phasen bewirkt. Diese beiden Phasen sind (i) die Schleppmittelphase, die geringe Mengen Methanol und DMC enthalten kann, und (ii) die Wasser enthaltende methanolische Phase, die geringe Mengen an DMC und Schleppmittel enthalten kann. Die Menge an erfindungsgemäß zuzusetzendem Wasser richtet sich grundsätzlich nach den Eigenschaften des Schleppmittels, insbesondere bezüglich seines Verhaltens bei der Phasentrennung. So führt ein Zusatz von weniger als 1 Gew.-% Wasser, bezogen auf das im Kopfprodukt vorhandene Methanol, im allgemeinen zu keiner oder zu einer unvollständigen Phasentrennung. Umgekehrt führt ein Zusatz von 10 000 Gew.-% Wasser, bezogen auf das im Kopfprodukt enthaltene Methanol, sehr wohl zu einer guten Phasentrennung, ein so hoher Wasserzusatz macht jedoch die Rückgewinnung des Methanols unwirtschaftlich. Der Wasserzusatz beträgt daher 1 bis 10 000 Gew.-%, bevorzugt 10 bis 1 000 Gew.-%, besonders bevorzugt 50 bis 500 Gew.-%, bezogen auf das im Gesamtkopfprodukt enthaltene Methanol.

Die nach der Phasentrennung erhaltene Schleppmittelphase, die nur geringe Mengen an den obengenannten weiteren Stoffen enthält, kann in dieser Form ohne weitere Reinigung in die Destillationskolonne zurückgeführt werden. In Abhängigkeit von der Natur des Schleppmittels kann diese Phase auch geringe Mengen an Wasser enthalten. Im allgemeinen ist dieser Wassergehalt jedoch wegen der erfindungsgemäß geforderten Nichtmischbarkeit des Schleppmittels mit Wasser äußerst klein, beispielsweise kleiner als 0,05 Gew.-%, bezogen auf die Menge des Schleppmittels. Sollte die Schleppmittelphase größere Anteile an Wasser, beispielsweise 0,5 Gew.-% oder mehr, enthalten, ist es möglich, die Schleppmittelphase nach der Trennung und vor der Rückführung in die Destillationskolonne zu trocknen, wozu beispiels-

EP 0 581 115 B1

weise Zeolithe eingesetzt werden können oder eine Azeotrop-Destillation durchgeführt werden kann. In bevorzugter Weise wird das Schleppmittel jedoch ohne vorherige Trocknung zurückgeführt.

Führt man das erfindungsgemäße Verfahren beispielsweise als diskontinuierliche Batchdestillation durch, so berechnet sich die Schleppmittelmenge p in kg wie folgt, wobei gilt:

1) Zusammensetzung des Gemisches DMC/CH$_3$OH:

$$x \text{ Gew.-\% DMC} + y \text{ Gew.-\% CH}_3\text{OH} = 100 \text{ Gew.-\%}$$

$$\text{Einsatzmenge des Gemisches in kg} = z.$$

2) Aus physikalischen Tabellenwerken entnommene oder ermittelte Zusammensetzung des Azeotrops aus Schleppmittel (S) und CH$_3$OH

$$n \text{ Gew.-\% S} + m \text{ Gew.-\% CH}_3\text{OH} = 100 \text{ Gew.-\%}.$$

Damit ergibt sich die Schleppmittelmenge p zu:

$$p[kg] = \frac{z \cdot y}{m} - \frac{z \cdot y}{100}$$

Will man beispielsweise 1150 kg eines Gemisches aus 41 Gew.-% DMC und 59 Gew.-% CH$_3$OH durch eine Batchdestillation mit Cyclohexan trennen, wobei die Zusammensetzung des Azeotrops aus Cyclohexan und CH$_3$OH zu 62 Gew.-% Cyclohexan und 38 Gew.-% CH$_3$OH ermittelt wurde, so ergibt sich für die Cyclohexanmenge p:

$$p \text{ (Cyclohexan)} = \frac{1150 \cdot 59}{38} - \frac{1150 \cdot 59}{100} = 1107 \text{ kg}$$

Diese optimale Menge an Schleppmittel ist unschwer zu berechnen, da die erforderlichen Daten leicht ermittelt werden können. Von der gemäß Rechenbeispiel erhaltenen optimalen Menge an Schleppmittel kann sowohl nach oben als auch nach unten abgewichen werden. Eine Unterschreitung bringt im allgemeinen jedoch keine Vorteile, eine Überschreitung kann in Bezug auf eine weitergehende Methanolabtrennung günstig sein. Erfindungsgemäß wird daher das 0,5- bis 3-fache der berechneten Menge p, bevorzugt das 0,8- bis 2-fache und besonders bevorzugt das 0,9- bis 1,5-fache dieser Menge p zugegeben.

Der besondere Vorteil des erfindungsgemäßen Verfahrens liegt darin, daß sich das durch die Azeotrop-Destillation erhaltene Kopfprodukt durch den beschriebenen Wasserzusatz im wesentlichen vollständig in zwei Phasen trennen läßt. Das Schleppmittel kann danach praktisch ohne Verlust in die Azeotrop-Destillation zurückgeführt werden. Dies ist in vorteilhaftem Gegensatz zu Verfahren des Standes der Technik, bei denen hohe Gehalte an Schleppmittel in der Methanolphase auftreten und entweder einen Verlust darstellen oder nur aufwendig zurückgewonnen werden können. Man benötigt daher bei einer kontinuierlich durchgeführten erfindungsgemäßen Destillation nur soviel Schleppmittel, wie in Abhängigkeit von der Natur des Schleppmittels bzw. von der Zusammensetzung des Azeotrops Schleppmittel/CH$_3$OH zur Aufrechterhaltung dieser azeotropen Zusammensetzung nötig ist. Die Schleppmittelmenge hängt damit im wesentlichen von apparativen Voraussetzungen, wie der Größe der Anlage und dem Hold up der Kolonnen, Rohre usw., ab; die Schleppmittelmenge ist daher nicht unmittelbar an die Einsatzmenge des zu trennenden DMC/Methanols gebunden, sondern hängt nur von der in jedem Zeitpunkt in der Destillationsapparatur befindlichen Menge dieses Gemisches ab.

Die wäßrig-methanolische Phase wird ebenfalls abgeführt und erfindungsgemäß destillativ in Methanol und ein wäßriges Bodenprodukt getrennt. Diese destillative Trennung CH$_3$OH/H$_2$O ist ein technisch üblicher und häufig angewandter Prozeß, der dem Fachmann geläufig ist. Das auf diese Weise gewonnene CH$_3$OH wird dabei als Kopfprodukt erhalten und kann direkt in die erneute Synthese von Dimethylcarbonat zurückgeführt werden. Eine geringe noch vorhandene Menge an Schleppmittel bzw. DMC stört dabei nicht. Es ist weiterhin möglich, aus der wäßrig-methanolischen Phase oder einem Teil davon zunächst eine Leichtsiederphase abzutrennen, die aus Schleppmittel, DMC und Methanol besteht, woraufhin nach Entnahme dieses Vorlaufs reines Methanol gewonnen wird. In bevorzugter Weise wird jedoch ohne Abtrennung eines solchen Leichtsieder-Vorlaufs gearbeitet und die gesamte Kopffraktion aus der CH$_3$OH/H$_2$O-Trennung in die DMC-Herstellung zurückgeführt.

Dieses erfindungsgemäße Verfahren ermöglicht gegenüber Verfahren des Standes der Technik eine nahezu vollständige Trennung des Schleppmittels und des Methanols in zwei genau abgegrenzte Phasen. Dadurch werden große Kreisströme, etwa an azeotropen Gemischen aus Schleppmittel/CH$_3$OH, vermieden, Dies stellt gegenüber dem Stand der Technik einen erheblichen Fortschritt dar.

Es ist weiterhin ein großer Vorteil des erfindungsgemäßen Verfahrens, daß auch Schleppmittel eingesetzt werden können, die mit Methanol allein mischbar sind und die somit ohne Wasserzusatz ein einphasiges Kopfprodukt der Azeotrop-Destillation ergeben. Es ist ausgesprochen überraschend, daß gerade unter diesen Schleppmitteln Beispiele gefunden werden, in denen im Kopfprodukt aus Schleppmittel und Methanol außergewöhnlich geringe Mengen an DMC enthalten sind, so daß die anschließende erfindungsgemäße Trennung mit Hilfe von Wasser ein nahezu DMC-freies Methanol liefert. Diesem Umstand ist es zu verdanken, daß auf die oben beschriebene Entnahme eines Leichtsieder-Vorlaufs verzichtet werden kann. Auch dadurch werden Kreisströme unnötig oder mindestens so stark reduziert, daß das erfindungsgemäße Verfahren äußerst kostengünstig durchgeführt werden kann.

Die folgenden Beispiele verdeutlichen das erfindungsgemäße Verfahren, ohne es jedoch auf diese Beispiele einzuschränken.

Beispiele

Beispiel 1 (zum Vergleich)

100 Gew.-Teile eines Gemisches aus 62,5 Gew.-% Methanol und 37,5 Gew.-% Dimethylcarbonat wurden mit 177,8 Gew.-Teilen (1,02 p) n-Hexan (z = 100; y = 62,5; m = 26,4) versetzt und einer azeotropen Destillation unterworfen. Man benutzte dazu eine verspiegelte 120 cm lange Füllkörperkolonne, die mit 4 x 4 mm Wilson-Spiralen gefüllt war. Das Rücklaufverhältnis (Rücklauf : Abnahme) betrug 5:1. Der Destillationsdruck war der aktuelle Umgebungsdruck. Am Siedepunkt von 50-51°C ging ein Hauptdestillat von 243,5 Gew.-Teilen über, danach folgte ein Zwischenlauf (Siedepunkt 51-90,5°C) von 4,0 Gew.-Teilen. Als Sumpfrückstand erhielt man 29,0 Gew.-Teile. Das Hauptdestillat wurde bei 0°C in zwei Phasen getrennt. Man erhielt 163,5 Gew.-Teile an einer Oberphase und 80,0 Gew.-Teile an einer Unterphase. Die Zusammensetzung der Phasen wurde mittels geeichter Gaschromatographie (GC) bestimmt.

Oberphase    95,0 % n-Hexan
             4,2 % Methanol
             0,8 % Dimethylcarbonat

Unterphase   29,8 % n-Hexan
             67,5 % Methanol
             2,7 % Dimethylcarbonat

Der Zwischenlauf hatte folgende GC-Zusammensetzung:

Zwischenlauf    71,1 % n-Hexan
                10,5 % Methanol
                18,4 % Dimethylcarbonat

Der Sumpfrückstand hatte folgende GC-Zusammensetzung:

Sumpfrückstand  0,07 % n-Hexan
                0,30 % Methanol
                99,63 % Dimethylcarbonat

Beispiel 2

Das Verfahren des Beispiels 1 wurde in allen Einzelheiten wiederholt. In das Hauptdestillat von 243,5 Gew.-Teilen wurden nun aber 62 Gew.-Teile Wasser (ca. 100 %, bezogen auf Methanol) gegeben. Danach wurden bei 20°C die Phasen getrennt. Man erhielt 179,0 Gew.-Teile an einer Oberphase und 125,5 Gew.-Teile einer Unterphase, deren GC-Zusammensetzung wie folgt war (Wasser wurde im GC nicht angezeigt):

Oberphase   99,61 % n-Hexan
            0,07 % Methanol
            0,32 % Dimethylcarbonat

Der Wassergehalt der Oberphase betrug laut Karl-Fischer-Titration 0,02 bis 0,03 Gew.-%.

Unterphase   0,16 % n-Hexan
             96,46 % Methanol
             3,38 % Dimethylcarbonat

Die Unterphase enthielt zusätzlich praktisch das gesamte zugegebene Wasser von ca. 62 Gew.-Teilen. Zwischenlauf und Sumpfrückstand hatten die gleiche Zusammensetzung wie in Beispiel 1.

Beispiel 3 (zum Vergleich)

Analog zu Beispiel 1 wurden 100 Gew.-Teile eines Gemisches aus 62,0 Gew.-% Methanol und 38,0 Gew.-% Dimethylcarbonat mit 66,5 Gew.-Teilen (1,13 p) n-Heptan (z = 100; y = 62,0; m = 51,5) versetzt und, wie dort angegeben, destilliert. Am Siedepunkt von 58 bis 59°C ging ein Hauptdestillat von 140,0 Gew.-Teilen über, danach folgte ein Zwischenlauf (Siedepunkt 60 bis 90,5°C) von 10,5 Gew.-Teilen. Als Sumpfrückstand erhielt man 15,0 Gew.-Teile. Das Hauptdestillat wurde bei 20°C in zwei Phasen getrennt. Man erhielt 40,0 Gew.-Teile an einer Oberphase und 99,5 Gew.-Teile an einer Unterphase. Die Zusammensetzung der Phasen wurde mittels geeichter Gaschromatographie bestimmt.

Oberphase    90,0 % n-Heptan
             5,4 % Methanol
             4,6 % Dimethylcarbonat

Unterphase   28,8 % n-Heptan
             58,9 % Methanol
             12,3 % Dimethylcarbonat

Der Zwischenlauf hatte folgende GC-Zusammensetzung:

Zwischenlauf   23,4 % n-Heptan
               8,4 % Methanol
               68,2 % Dimethylcarbonat

Der Sumpfrückstand hatte folgende GC-Zusammensetzung:

Sumpfrückstand   0,04 % n-Heptan
                 0,05 % Methanol
                 99,91 % Dimethylcarbonat

Beispiel 4

Das Verfahren des Beispiels 3 wurde in allen Einzelheiten wiederholte In das Hauptdestillat von 140,0 Gew.-Teilen wurden nun aber 62 Gew.-Teile Wasser (ca. 100 %, bezogen auf Methanol) gegeben. Danach wurden bei 20°C die Phasen getrennt. Man erhielt 66,0 Gew.-Teile an einer Oberphase und 137,5 Gew.-Teile an einer Unterphase, deren GC-Zusammensetzung wie folgt war (Wasser wurde im GC nicht angezeigt):

Oberphase   98,9 % n-Heptan
            0,1 % Methanol
            1,0 % Dimethylcarbonat

Der Wassergehalt der Oberphase betrug laut Karl-Fischer-Titration 0,02 Gew.-%.

Unterphase   0,3 % n-Heptan
             84,0 % Methanol
             15,7 % Dimethylcarbonat

Die Unterphase enthielt zusätzlich praktisch das gesamte zugegebene Wasser von ca. 62 Gew.-Teilen. Zwischenlauf und Sumpfrückstand hatten die gleiche Zusammensetzung wie in Beispiel 3.

Beispiel 5 (zum Vergleich)

Analog zu Beispiel 1 wurden 100 Gew.-Teile eines Gemisches aus 65,5 Gew.-% Methanol und 34,5 Gew.-% Dimethylcarbonat mit 66,7 Gew.-Teilen (1,15 p) Isooctan (2,4,4-Trimethylpentan; z = 100; y = 65,5; m = 53,0) gemischt und, wie dort angegeben, destilliert. Am Siedepunkt von 59,0 bis 60°C ging ein Hauptdestillat von 141,4 Gew.-Teilen über, danach folgte ein Zwischenlauf (Siedepunkt 60 bis 90,5°C) von 11,3 Gew.-Teilen. Als Sumpfrückstand erhielt man 16,6 Gew.-Teile. Das Hauptdestillat wurde bei 28°C in zwei Phasen getrennt. Man erhielt 40,9 Gew.-Teile an einer Oberphase und 100,0 Gew.-Teile an einer Unterphase. Die Zusammensetzung der Phasen wurde mittels geeichter Gaschromatographie bestimmt.

Oberphase    89,3 % Isooctan
             7,9 % Methanol
             2,8 % Dimethylcarbonat

Unterphase   31,2 % Isooctan
             58,5 % Methanol
             10,3 % Dimethylcarbonat

Der Zwischenlauf hatte folgende GC-Zusammensetzung

Zwischenlauf    25,5 % Isooctan
                34,2 % Methanol
                40,3 % Dimethylcarbonat

Der Sumpfrückstand hatte folgende GC-Zusammensetzung

Sumpfrückstand   kein Isooctan
                 0,80 % Methanol
                 99,20 % Dimethylcarbonat

Beispiel 6

Das Verfahren des Beispiels 5 wurde in allen Einzelheiten wiederholt. In das Hauptdestillat von 141,4 Gew.-Teilen wurden nun aber 128 Gew.-Teile Wasser (ca. 200 %, bezogen auf Methanol) gegeben. Danach wurden bei 30°C die Phasen getrennt. Man erhielt 65,8 Gew.- Teile an einer Oberphase und 202,8 Gew.-Teile an einer Unterphase, deren GC-Zusammensetzung wie folgt war (Wasser wurde im GC nicht angezeigt).

Oberphase   99,59 % Isooctan
            0,02 % Methanol
            0,39 % Dimethylcarbonat

Der Wassergehalt der Oberphase betrug laut Karl-Fischer-Titration 0,02 bis 0,03 Gew.-%.

Unterphase   0,04 % Isooctan
             87,10 % Methanol
             12,86 % Dimethylcarbonat

Die Unterphase enthielt zusätzlich praktisch das gesamte zugegebene Wasser von ca. 128 Gew.-Teilen. Zwischenlauf und Sumpfrückstand hatten die gleiche Zusammensetzung wie in Beispiel 5.

Beispiel 7

Analog zu Beispiel 1 wurden 100 Gew.-Teile eines Gemisches aus 63,5 Gew.-% Methanol und 36,5 Gew.-% Dimethylcarbonat mit 150,0 Gew.-Teilen (1,06 p) 1-Chlorbutan (z = 100; y = 63,5; m = 28,5) gemischt und wie dort angegeben destilliert. Am Siedepunkt von 57,5 bis 58°C ging ein Hauptdestillat von 204,8 Gew.-Teilen über, danach folgte ein Zwischenlauf (Siedepunkt 58 bis 91°C) von 13,8 Gew.-Teilen. Als Sumpfrückstand erhielt man 29,7 Gew.-Teile. Das Hauptdestillat, das bis -20°C einphasig war, wurde mit 128 Gew.-Teilen Wasser (ca. 200 %, bezogen auf Methanol) versetzt und bei 25°C in zwei Phasen getrennt. Man erhielt 141,2 Gew.-Teile an einer Oberphase und 188,9 Gew.-Teile an einer Unterphase. Die Zusammensetzung der Phasen wurde mittels geeichter Gaschromatographie bestimmt (Wasser wurde im GC nicht angezeigt).

Oberphase   99,90 % 1-Chlorbutan
            kein Methanol
            0,10 % Dimethylcarbonat

Der Wassergehalt der Oberphase betrug laut Karl-Fischer-Titration 0,04 Gew.-%.

Unterphase    0,74 % 1-Chlorbutan
              99,17 % Methanol
              0,09 % Dimethylcarbonat

Die Unterphase enthielt zusätzlich praktisch das gesamte zugegebene Wasser von ca. 128 Gew.-Teilen.
Der Zwischenlauf hatte folgende GC-Zusammensetzung:

Zwischenlauf    52,1 % 1-Chlorbutan
                9,4 % Methanol
                38,5 % Dimethylcarbonat

Der Sumpfrückstand hatte folgende GC-Zusammensetzung:

Sumpfrückstand    0,06 % 1-Chlorbutan
                  0,62 % Methanol
                  99,32 % Dimethylcarbonat

Beispiel 8

Analog zu Beispiel 1 wurden 100 Gew.-Teile eines Gemisches aus 62,6 Gew.-% Methanol und 37,4 Gew.-% Dimethylcarbonat mit 113,0 Gew.-Teilen (1,16 p) Benzol (z = 100; y = 62,6; m = 39,1) gemischt und, wie dort angegeben, destilliert. Am Siedepunkt von 59 bis 59,5°C ging ein Hauptdestillat von 164,6 Gew.-Teilen über, danach folgte ein Zwischenlauf (Siedepunkt 60 bis 91°C) von 16,2 Gew.-Teilen. Als Sumpfrückstand erhielt man 30,8 Gew.-Teile. Das Hauptdestillat, das bei Raumtemperatur einphasig war und bei -20°C zum Teil erstarrte, wurde mit 125 Gew.-Teilen Wasser (ca. 200 %, bezogen auf Methanol) versetzt und bei 25°C in zwei Phasen getrennt. Man erhielt 101,0 Gew.-Teile an einer Oberphase und 187,3 Gew.-Teile an einer Unterphase. Die Zusammensetzung der Phasen wurde mittels geeichter Gaschromatographie bestimmt (Wasser wurde in GC nicht angezeigt).

Oberphase    98,56 % Benzol
             1,29 % Methanol
             0,15 % Dimethylcarbonat

Der Wassergehalt der Oberphase betrug laut Karl-Fischer-Titration 0,06 Gew.-%.

Unterphase    1,57 % Benzol
              98,39 % Methanol
              0,04 % Dimethylcarbonat

Die Unterphase enthielt zusätzlich praktisch das gesamte zugegebene Wasser von ca. 125 Gew.-Teilen.
Der Zwischenlauf hatte folgende GC-Zusammensetzung:

Zwischenlauf    61,57 % Benzol
                3,71 % Methanol
                34,72 % Dimethylcarbonat

Der Sumpfrückstand hatte folgende GC-Zusammensetzung:

Sumpfrückstand    0,03 % Benzol
                  0,87 % Methanol
                  99,10 % Dimethylcarbonat

Beispiel 9

Analog zu Beispiel 1 wurden 100 Gew.-Teile eines Gemisches aus 62,6 Gew.-% Methanol und 37,4 Gew.-% Dimethylcarbonat mit 146,3 Gew.-Teilen (1,10 p) Methylcyclopentan (z = 100; y = 62,6; m = 32,0) gemischt und, wie dort angegeben, jedoch bei einem Rücklaufverhältnis von 2:1, destilliert. Am Siedepunkt von 52 bis 52,5°C ging ein Hauptdestillat von 205,3 Gew.-Teilen über, danach folgte ein Zwischenlauf (Siedepunkt 52,5 bis 91°C) von 9,8 Gew.-Teilen. Als Sumpfrückstand erhielt man 29,7 Gew.-Teile. Das Hauptdestillat, das bei 35°C einphasig war, wurde mit 62,0 Gew.-Teilen Wasser (ca. 100 %, bezogen auf Methanol) versetzt und bei 35°C in zwei Phasen getrennt. Man erhielt 143,9

8

Gew.-Teile an einer Oberphase und 122,5 Gew.-Teile an einer Unterphase. Die Zusammensetzung der Phasen wurde mittels geeichter Gaschromatographie bestimmt (Wasser wurde im GC nicht angezeigt).

Oberphase    98,40 % Methylcyclopentan
            0,58 % Methanol
            1,02 % Dimethylcarbonat

Der Wassergehalt der Oberphase betrug laut Karl-Fischer-Titration 0,01 bis 0,02 Gew.-%.

Unterphase    0,40 % Methylcyclopentan
            96,21 % Methanol
            3,39 % Dimethylcarbonat

Die Unterphase enthielt zusätzlich praktisch das gesamte zugegebene Wasser von ca. 62 Gew.-Teilen.
Der Zwischenlauf hatte folgende GC-Zusammensetzung:

Zwischenlauf    23,5 % Methylcyclopentan
            40,2 % Methanol
            36,3 % Dimethylcarbonat

Der Sumpfrückstand hatte folgende GC-Zusammensetzung:

Sumpfrückstand    0,14 % Methylcyclopentan
            0,22 % Methanol
            99,64 % Dimethylcarbonat

Beispiel 10 (zum Vergleich)

Analog zu Beispiel 1 wurden 100 Gew.-Teile eines Gemisches aus 62,3 Gew.-% Methanol und 37,7 Gew.-% Dimethylcarbonat mit 111,8 Gew.-Teilen (1,10 p) Cyclohexan (z = 100; y = 62,3; m = 38) versetzt und, wie dort angegeben, jedoch bei einem Rücklaufverhältnis von 2:1, destilliert. Am Siedepunkt von 55,5 bis 56,5°C ging ein Hauptdestillat von 179,9 Gew.-Teilen über, danach folgte ein Zwischenlauf (Siedepunkt 56,5 bis 90,5°C) von 6,8 Gew.-Teilen. Als Sumpfrückstand erhielt man 24,2 Gew.-Teile. Das Hauptdestillat wurde bei 35°C in zwei Phasen getrennt. Man erhielt 83,4 Gew.-Teile an einer Oberphase und 96,0 Gew.-Teile an einer Unterphase. Die Zusammensetzung der Phasen wurde mittels geeichter Gaschromatographie bestimmt.

Oberphase    83,87 % Cyclohexan
            12,76 % Methanol
            3,37 % Dimethylcarbonat

Unterphase    45,10 % Cyclohexan
            49,23 % Methanol
            5,67 % Dimethylcarbonat

Der Zwischenlauf hatte folgende GC-Zusammensetzung:

Zwischenlauf    30,57 % Cyclohexan
            40,23 % Methanol
            29,20 % Dimethylcarbonat

Der Sumpfrückstand hatte folgende GC-Zusammensetzung:

Sumpfrückstand    0,04 % Cyclohexan
            0,34 % Methanol
            99,62 % Dimethylcarbonat

<u>Beispiel 11</u>

Das Verfahren des Beispiels 10 wurde in allen Einzelheiten wiederholt. In das Hauptdestillat von 179,9 Gew.-Teilen wurden nun aber 62,0 Gew.-Teile Wasser (ca. 100 %, bezogen auf Methanol) gegeben. Danach wurden bei 35°C die Phasen getrennt. Man erhielt 112,1 Gew.-Teile an einer Oberphase und 128,5 Gew.-Teile an einer Unterphase, deren GC-Zusammensetzung wie folgt war (Wasser wurde im GC nicht angezeigt):

Oberphase    97,43 % Cyclohexan
             0,54 % Methanol
             2,03 % Dimethylcarbonat

Der Wassergehalt der Oberphase betrug laut Karl-Fischer-Titration 0,02 Gew.-%.

Unterphase    0,49 % Cyclohexan
              92,03 % Methanol
              7,48 % Dimethylcarbonat

Die Unterphase enthielt zusätzlich praktisch das gesamte zugegebene Wasser von ca. 62 Gew.-Teilen. Zwischenlauf und Sumpfrückstand hatten die gleiche Zusammensetzung wie in Beispiel 10.

**Patentansprüche**

1. Verfahren zur Abtrennung von Methanol aus Dimethylcarbonat/Methanol-Gemischen durch Azeotrop-Destillation mit einem Schleppmittel, dadurch gekennzeichnet, daß

   a) ein mit Methanol ein Azeotrop bildendes Schleppmittel eingesetzt wird, das mit Wasser nicht mischbar ist,

   b) das Azeotrop dieses Schleppmittels mit Methanol bei tieferer Temperatur als das Azeotrop Dimethylcarbonat/Methanol siedet,

   c) das Azeotrop Schleppmittel/Methanol, gegebenenfalls mit geringen Mengen Dimethylcarbonat, mit Wasser zur Bildung zweier Phasen versetzt wird,

   d) die gebildeten Phasen getrennt werden und die Schleppmittelphase, die geringe Mengen an Methanol und Dimethylcarbonat enthalten kann, in die Destillationskolonne zurückgeführt und die wäßrig-methanolische Phase, die geringe Mengen an Dimethylcarbonat und Schleppmittel enthalten kann, durch Destillation auf Methanol und Dimethylcarbonat/Schleppmittel aufgearbeitet wird und

   e) aus dem Methanol-freien Sumpf der Azeotrop-Destillation das Dimethylcarbonat gewonnen wird.

2. Verfahren nach Anspruch 1, dadurch gekennzeichnet, daß als Schleppmittel eines oder mehrere aus den Stoffklassen der geradkettigen oder verzweigten gesättigten $C_5$-$C_8$-Kohlenwasserstoffe, der cyclischen $C_5$-$C_8$-Kohlenwasserstoffe, der geradkettigen oder verzweigten $C_5$-$C_8$-Kohlenwasserstoffe mit einer end- oder innenständigen Doppelbindung, des Benzols, der halogenierten $C_2$-$C_4$-Kohlenwasserstoffe, des Fluorbenzols, der offenkettigen oder cyclischen, gesättigten oder ungesättigten $C_4$-$C_8$-Ether oder Thioether und der $C_4$-$C_8$-Ester ausgewählt wird.

3. Verfahren nach Anspruch 2, dadurch gekennzeichnet, daß als Schleppmittel eines oder mehrere aus der Gruppe von n-Pentan, n-Hexan, n-Heptan, n-Octan und dessen verzweigten Isomeren, Cyclopentan, Cyclohexan, Methylcyclopentan, Ethylcyclobutan, Cycloheptan, Methylcyclohexan, Ethylcyclopentan, Propylcyclobutan, n-Penten, n-Hexen, n-Hepten, n-Octen sowie deren verzweigten Isomeren und deren Isomeren mit end- bzw. innenständiger Doppelbindung, Benzol, 1,1,1- Trichlorethan, 1,2-Dichlorethan, 1,2-Dichlor-1-propen, 1-Brompropan, 1-Chlorbutan, 2-Brom-2-methylpropan, 1-Chlor-3-methylbutan, 1,1,2-Trichlor-trifluoroethan, Fluorbenzol, Butylmethylether, Methyl-tert.-butyl-ether, Diisopropylether, Furan, 2-Methylfuran, 2,5-Dimethylfuran, Thiophen, Propylformiat, Isopropylformiat, Methylpropionat auswählt.

4. Verfahren nach Anspruch 1, dadurch gekennzeichnet, daß 30 bis 300 Gew.-% Schleppmittel, bezogen auf das abzutrennende Methanol, eingesetzt werden.

**5.** Verfahren nach Anspruch 1, dadurch gekennzeichnet, daß 1 bis 10 000 Gew.-%, bevorzugt 10 bis 1000 Gew.-%, besonders bevorzugt 50 bis 500 Gew.-% Wasser, bezogen auf das im Azeotrop enthaltene Methanol, eingesetzt werden.

**6.** Verfahren nach Anspruch 1, dadurch gekennzeichnet, daß das zurückzuführende Schleppmittel keiner Trocknung unterworfen wird.

**7.** Verfahren nach Anspruch 1, dadurch gekennzeichnet, daß das aus der wäßrig-methanolischen Phase durch Destillation gewonnene Methanol, das kleine Mengen an Schleppmittel und an Dimethylcarbonat enthalten kann, direkt in die Herstellung von Dimethylcarbonat zurückgeführt wird.

**Claims**

**1.** Process for separating methanol from dimethyl carbonate/methanol mixtures by azeotropic distillation using an entraining agent, characterised in that

a) an entraining agent that forms an azeotrope with methanol and that is immiscible with water is used,

b) the azeotrope of this entraining agent with methanol boils at a lower temperature than the dimethyl carbonate/methanol azeotrope,

c) the entraining agent/methanol azeotrope, which may or may not contain small amounts of dimethyl carbonate, is mixed with water to form two phases,

d) the phases formed are separated and the entraining agent phase, which may contain small amounts of methanol and dimethyl carbonate, is returned to the distillation column and the aqueous-methanol phase, which may contain small amounts of dimethyl carbonate and entraining agent, is worked up into methanol and dimethyl carbonate/entraining agent by distillation, and

e) the dimethyl carbonate is recovered from the methanol-free product from the bottom of the azeotropic distillation column.

**2.** Process according to Claim 1, characterised in that the entraining agent is chosen from one or more of the classes of substances comprising straight-chain or branched saturated $C_5$-$C_8$-hydrocarbons, cyclic $C_5$-$C_8$-hydrocarbons, straight-chain or branched $C_5$-$C_8$-hydrocarbons having a terminal or internal double bond, benzene, halogenated $C_2$-$C_4$-hydrocarbons, fluorobenzene, open-chain or cyclic, saturated or unsaturated $C_4$-$C_8$-ethers or -thioethers, and $C_4$-$C_8$-esters.

**3.** Process according to Claim 2, characterised in that the entraining agent is chosen from one or more of the group comprising n-pentane, n-hexane, n-heptane, n-octane and their branched isomers, cyclopentane, cyclohexane, methylcyclopentane, ethylcyclobutane, cycloheptane, methylcyclohexane, ethylcyclopentane, propylcyclobutane, n-pentene, n-hexene, n-heptene, n-octene, and also their branched isomers and their isomers having a terminal or internal double bond, benzene, 1,1,1-trichloroethane, 1,2-dichloroethane, 1,2-dichloro-1-propene, 1-bromopropane, 1-chlorobutane, 2-bromo-2-methylpropane, 1-chloro-3-methylbutane, 1,1,2-trichloro-trifluoroethane, fluorobenzene, butyl methyl ether, methyl tert-butyl ether, diisopropyl ether, furan, 2-methylfuran, 2,5-dimethylfuran, thiophene, propyl formate, isopropyl formate, methyl propionate.

**4.** Process according to Claim 1, characterised in that from 30 to 300% by weight of entraining agent, based on the methanol to be separated, are used.

**5.** Process according to Claim 1, characterised in that from 1 to 10,000% by weight, preferably from 10 to 1,000% by weight, particularly preferably from 50 to 500% by weight of water, based on the methanol contained in the azeotrope, are used.

**6.** Process according to Claim 1, characterised in that the entraining agent to be recycled is not subjected to drying.

**7.** Process according to Claim 1, characterised in that the methanol obtained from the aqueous-methanolic phase by distillation, which may contain small amounts of entraining agent and dimethyl carbonate, is returned directly to the preparation of dimethyl carbonate.

**Revendications**

1. Procédé pour la séparation de méthanol de mélanges de carbonate de diméthyle et de méthanol par distillation azéotrope avec un agent d'entraînement, caractérisé en ce que

   a) on utilise un agent d'entraînement formant un mélange azéotrope avec le méthanol et qui n'est pas miscible avec l'eau,
   b) le mélange azéotrope de cet agent d'entraînement avec le méthanol bout à température plus basse que le mélange azéotrope carbonate de diméthyle/méthanol,
   c) le mélange azéotrope agent d'entraînement/méthanol, comportant éventuellement de petites quantités de carbonate de diméthyle, est additionné d'eau pour formation de deux phases,
   d) les phases formées sont séparées, et la phase de l'agent d'entraînement, qui peut contenir de petites quantités de méthanol et de carbonate de diméthyle, est renvoyée dans la colonne de distillation, et la phase eau-méthanol, qui peut contenir de petites quantités de carbonate de diméthyle et d'agent d'entraînement, est traitée par distillation pour donner du méthanol et un mélange de carbonate de diméthyle et d'agent d'entraînement, et
   e) le carbonate de diméthyle est récupéré dans le fond, dépourvu de méthanol, de la distillation azéotrope.

2. Procédé selon la revendication 1, caractérisé en ce que l'on sélectionne comme agent d'entraînement un ou plusieurs agents d'entraînement des classes des hydrocarbures saturés à chaîne linéaire ou ramifiée en $C_5$-$C_8$, des hydrocarbures cycliques en $C_5$-$C_8$, des hydrocarbures à chaîne linéaire ou ramifiée en $C_5$-$C_8$ présentant une double liaison terminale ou intermédiaire, du benzène, des hydrocarbures halogénés en $C_2$-$C_4$, du fluorobenzène, des éthers ou thioéthers à chaîne ouverte ou cyclique, saturés ou insaturés en $C_4$-$C_8$ et des esters en $C_4$-$C_8$.

3. Procédé selon la revendication 2, caractérisé en ce que l'on sélectionne comme agent d'entraînement un ou plusieurs des membres du groupe constitué du n-pentane, du n-hexane, du n-heptane, du n-octane et de leurs isomères ramifiés, du cyclopentane, du cyclohexane, du méthylcyclopentane, de l'éthylcyclobutane, du cycloheptane, du méthylcyclohexane, de l'éthylcyclopentane, du propylcyclobutane, du n-pentène, du n-hexène, du n-heptène, du n-octène ainsi que leurs isomères ramifiés et leurs isomères présentant une double liaison terminale ou intermédiaire, du benzène, du 1,1,1-trichloroéthane, du 1,2-dichloroéthane, du 1,2-dichloro-1-propène, du 1-bromopropane, du 1-chlorobutane, du 2-bromo-2-méthylpropane, du 1-chloro-3-méthylbutane, du 1,1,2-trichloro-trifluoroéthane, du fluorobenzène, du butylméthyléther, du méthyl-terr-butyléther, du diisopropyléther, du furane, du 2-méthylfurane, du 2,5-diméthylfurane, du thiophène, du formiate de propyle, du formiate d'isopropyle et du propionate de méthyle.

4. Procédé selon la revendication 1, caractérisé en ce que l'on utilise de 30 à 300% en poids d'agents d'entraînement, calculés sur le méthanol à séparer.

5. Procédé selon la revendication 1, caractérisé en ce que l'on utilise de 1 à 10000% en poids, de préférence de 10 à 1000% en poids, et de manière particulièrement préférée de 50 à 500% en poids d'eau, calculés sur le méthanol contenu dans le mélange azéotrope.

6. Procédé selon la revendication 1, caractérisé en ce que l'agent d'entraînement à récupérer n'est pas soumis à un séchage.

7. Procédé selon la revendication 1, caractérisé en ce que le méthanol récupéré par distillation de la phase eau-méthanol, qui peut contenir de petites quantités d'agent d'entraînement et de carbonate de diméthyle, est renvoyée directement dans la préparation de carbonate de diméthyle.